# EUROPEAN PATENT SPECIFICATION

(11) **EP 0 517 777 B1**
(45) Date of publication and mention of the grant of the patent: **22.05.1996**
(21) Application number: 91905365.2
(22) Date of filing: 04.03.1991
(51) Int. Cl.: G01N 21/75, G01N 33/543

(54) **SAMPLE CELL FOR USE IN CHEMICAL OR BIOCHEMICAL ASSAYS**
MESSZELLE FÜR CHEMISCHE ODER BIOCHEMISCHE PROBEN
CELLULE ECHANTILLON POUR UTILISATION DANS LES ANALYSES CHIMIQUES OU BIOLOGIQUES

(30) Priority: 02.03.1990 GB 9004672; 07.03.1990 GB 9005154
(43) Date of publication of application: 16.12.1992
(73) Proprietor: FISONS plc, Ipswich Suffolk IP1 1QH (GB)
(72) Inventor: FORTUNE, David, Harry, Codicote, Hertfordshire SG4 8UU (GB); MOLLOY, James, Oscar, Belmont, Surrey SM2 6BP (GB); MAULE, Colin, Hugh, Cambridge CB4 1QG (GB)
(74) Representative: Jones, Stephen Anthony
(86) International application number: GB9100330
(87) International publication number: WO9113339

(56) References cited:
- EP-A- 0 257 955
- EP-A- 0 305 109
- WO-A-84/02578
- US-A- 4 877 747

## Description

This invention relates to optical biosensors for determining analytes in solution, in particular to resonant optical biosensors.

Many devices for the automatic determination of biochemical analytes in solution have been proposed in recent years. Typically, such devices (biosensors) include a sensitised coating layer which is located in the evanescent region of a resonant field. Detection of the analyte typically utilizes optical techniques such as, for example, surface plasmon resonance, and is based on changes in the thickness and/or refractive index of the coating layer resulting from interaction of that layer with the analyte. This causes a change, eg in the angular position of the resonance.

One of the techniques which has been suggested for use in optical biosensors is frustrated total reflection. The principles of frustrated total reflection (FTR) are well-known; the technique is described, for example, by Bosacchi and Oehrle [Applied Optics (1982), 21, 2167-2173]. An FTR device for use in immunoassay is disclosed in US Patent 4,857,273 and comprises a cavity layer bounded on one side by the sample under investigation and on the other side by a spacer layer which in turn is mounted on a substrate. The substrate-spacer layer interface is irradiated with monochromatic radiation such that total reflection occurs, the associated evanescent field penetrating through the spacer layer. If the thickness of the spacer layer is correct and the incident parallel wave vector matches one of the resonant mode propagation constants, the total reflection is frustrated and radiation is coupled into the cavity layer. The cavity layer must be composed of material which has a higher refractive index than the spacer layer and which is transparent at the wavelength of the incident radiation.

More recently, FTR biosensors have been described [see, for example, PCT Patent Application WO90/06503] in which the cavity layer is a thin film of relatively high refractive index material. Particularly in such cases it is generally desirable, for ease of handling, that the cavity layer be supported on a substrate, eg a glass chip or slide.

A problem which is encountered with devices of this kind is related to the need to couple the exciting radiation into the substrate. This is conventionally performed by mounting the substrate on a glass prism or lens to which an appropriate thin layer of index-matching coupling agent, such as oil, water or glycerol, has been applied. Such devices are disclosed in, for example, British Patent Application No. 2197065 and European Patent Application No. 305109. Clearly, for routine applications the necessity of applying such a fluid to the surface of the prism before placing the substrate in position is a considerable disadvantage which considerably increases the chances of mistakes being made in the measurement, either through misuse of the apparatus or as a result of contamination of the oil, and hence of erroneous results being obtained. Also, the level of skill required of the operator of the device is much greater than would otherwise be the case.

We have now devised an improved form of resonant optical biosensor which overcomes or substantially mitigates the above disadvantages.

Thus according to the invention there is provided a sample cell for use in the qualitative or quantitative determination of an analyte, according to claim 1.

The device according to the invention is advantageous inter alia in that positioning of the sample cell in the detection apparatus does not require the application of an index-matching agent such as oil. The ease of use of the apparatus is thereby greatly increased and the potential for errors occurring as a result of misuse correspondingly reduced. The elimination of the requirement for the use an index-matching agent is also advantageous in that such materials are commonly toxic.

It is preferred that the resonant optical biosensor be accommodated at an upper portion of the transparent block, the optical coupling means being formed integrally in a lower portion of the block. By "accommodated" is not necessarily meant that the biosensor is enclosed within the block or located in a well therein; the biosensor may, for example, be formed on a generally flat surface of the block. Similarly, by "sample cell" is not necessarily meant a container or well which holds a sample under test; the cell could, for example have a generally flat top surface to which a drop of sample is applied.

For routine applications it is envisaged that the sample cell would be a disposable unit, the cell being easily mounted on the detection apparatus and removed and discarded after use.

The transparent block may be of any suitable material which is transparent to the incident and reflected radiation ie which shows little or no absorptivity at the wavelength(s) of the incident radiation. For ease of manufacture, the preferred transparent materials are clear plastics. Suitable such plastics materials will be readily apparent to those skilled in the art.

The resonant optical biosensor will generally comprise
a) a cavity layer of transmissive dielectric material of refractive index n₃,
b) a dielectric substrate of refractive index n₁, and
c) interposed between the cavity layer and the substrate, a dielectric spacer layer of refractive index n₂,
the arrangement being such that, when the interface between the substrate and the spacer layer is irradiated with light such that total reflection occurs, the total reflection may be frustrated by the propagation of a resonant guided mode within the cavity layer.

In this context, 'light' may include not only visible light but also wavelengths above and below this range, eg in the ultraviolet and infra-red.

Resonant propagation of a guided mode in the cavity layer will occur, for a given wavelength, at a particular angle of incidence of the exciting radiation. Thus, two basic measurement approaches are possible: scanning the angle of incidence at fixed wavelength or scanning the wavelength at a fixed angle of incidence. The former approach, using monochromatic radiation, is preferred since it allows the use of a laser source, simplifying the problem of optical collimation, and avoids dispersion effects, thereby simplifying the analysis of the results.

The angular position of the resonant effect depends on various parameters of the biosensor device, such as the refractive indices and thicknesses of the various layers. In general, it is a pre-requisite that the refractive index n₃ of the cavity layer and the refractive index n₁ of the substrate should both exceed the refractive index n₂ of the spacer layer. Also, since at least one mode must exist in the cavity to achieve resonance, the cavity layer must exceed a certain minimum thickness.

The cavity layer is preferably a thin-film of dielectric material. Suitably transmissive dielectric materials for the cavity layer include zirconium dioxide, titanium dioxide, aluminium oxide and tantalum oxide.

The cavity layer may be prepared by known techniques, eg vacuum evaporation, sputtering, chemical vapour deposition or indiffusion.

The dielectric spacer layer must also be suitably transmissive to the incident radiation and must have a lower refractive index than both the cavity layer and the substrate. The layer may, for example, comprise an evaporated or sputtered layer of magnesium fluoride. In this case an infra-red light injection laser may be used as light source. The light from such a source typically has a wavelength around 800 nm. Other suitable materials include lithium fluoride and silicon dioxide. Apart from the evaporation and sputtering techniques mentioned above, the spacer layer may be deposited on the substrate by a sol-gel process, or be formed by chemical reaction with the substrate.

The sol-gel process is particularly preferred where the spacer layer is of silicon dioxide.

The refractive index of the substrate (n₁) must be greater than that (n₂) of the spacer layer but the thickness of the substrate is generally not critical to the performance of the invention.

By contrast, the thickness of the cavity layer must be so chosen that resonance occurs within an appropriate range of coupling angles. The spacer layer will typically have a thickness of the order of several hundred nanometres, say from about 200nm to 2000nm, more preferably 500 to 1500nm, eg 1000nm. The cavity layer typically has a thickness of a few tens of nanometres, say 10 to 200nm, more preferably 30 to 150nm, eg 100nm.

It is particularly preferred that the cavity layer has a thickness of 30 to 150nm and comprises a material selected from zirconium dioxide, titanium dioxide, tantalum oxide and aluminium oxide, and the spacer layer has a thickness of 500 to 1500nm and comprises a material selected from magnesium fluoride, lithium fluoride and silicon dioxide, the choice of materials being such that the refractive index of the spacer layer is less than that of the cavity layer.

Preferred materials for the cavity layer and the spacer layer are tantalum oxide and silicon dioxide respectively.

The cavity layer is generally sensitised by having biomolecules, eg specific binding partners for the analytes under test, immobilised upon it. Suitable such binding partners and methods for their immobilisation will be apparent to those skilled in the art. Each cavity layer may carry more than one sensitised area and each such area may be sensitised to the same analyte or different analytes.

One form of optical coupling means which may be formed integrally in the transparent block is a grating.

The grating may be formed in a surface of the substrate opposite to that adjacent the spacer layer, or in an additional component bonded to that surface. The grating may, for example, be formed in a thin adhesive layer affixed to that opposite surface. In this case, the substrate may constitute, or be part of, the transparent block.

Alternatively, the grating may be formed in the external surface of a separate transparent block to which the substrate is affixed. In this case, the transparent block is preferably a moulded article, eg of plastics material.

An alternative, and preferred, form of optical coupling means is a prism. The prism preferably forms part of a moulded article, eg of plastics. The prism may have any conventional form, eg triangular, trapezoidal, rectangular or hemicylindrical.

A sample well formed in the transparent block may act as a metering chamber. Ducts may be formed in the device along which sample may be conveyed to the sample well. Flow of the sample into the well may be facilitated by appropriate surface treatment, eg coating with a wetting agent.

Where the resonant optical biosensor is accommodated within or on a moulded article, the substrate of the biosensor may be a chip of, for example, glass. This is preferably bonded to the moulded article, for instance at the base of a sample well formed therein, by means of a refractive index matching adhesive or fluid.

As an alternative, the moulded article may itself constitute the substrate of the biosensor, the spacer layer and the cavity layer being deposited directly on the surface of the moulded article. This arrangement has the advantages of simplicity of manufacture and complete elimination of any need for potentially harmful index-matching agents.

As well as accommodating the resonant optical biosensor, the moulded article may include features defining a sample well and may be appropriately shaped to facilitate handling.

In a particularly preferred embodiment the sample cell comprises a moulded transparent block upon which are coated the spacer layer and cavity layer of a resonant optical biosensor, the block fitting (eg by a snap-fit) a housing in which is formed a sample entry port, a sample chamber or duct being defined between the block and the housing.

Blocks, eg prisms, for coating with the spacer layer and the cavity layer are preferably moulded as a wafer comprising a plurality of such blocks frangibly connected at their edges. The layers are preferably applied to the surface of the wafer and the individual blocks then separated.

The sample cell according to the invention will generally form part of, or be used in association with, an apparatus comprising a light source and suitable detection means. The sample cell may be located in an aperture in the housing of such apparatus. Suitable keying means may be provided to ensure proper positioning of the sample cell.

Any convenient source of radiation may be used as the source of the incident light but, as noted above, it is preferable to use monochromatic radiation and the most convenient source of such radiation is a laser. The choice of laser will depend inter alia on the materials used for the various layers of which some examples have already been given.

The scanning of angle may be performed either sequentially or simultaneously ie by varying the angle of incidence of a parallel beam of light or by simultaneously irradiating over a range of angles using a fan-shaped beam of light as described (in connection with surface plasmon resonance) in European Patent Application No. 0305109A. In the former case, a single-channel detector may be used which is mechanically scanned over a range of angles; in the latter case, in which a range of angles is irradiated simultaneously, it will generally be necessary to use a multi-channel detector having angular resolution.

At resonance, the incident light is coupled into the cavity layer by FTR, propagates a certain distance along the cavity layer, and couples back out (also by FTR). The propagation distance depends on the various device parameters but is typically of the order of 1 or 2mm.

In general, at resonance, the reflected light will undergo a phase change and it may be the angular position at which this phase change occurs which is detected.

Changes on the surface of the cavity layer, eg binding of antigen to immobilised antibody, cause changes in the thickness of the layer of immobilised biochemicals and hence shift the angular position of the resonance.

In some formats, there may also be a reduction in the intensity of the reflected light, eg if the immobilised species are absorbing at the wavelength of the incident radiation. In this case, this reduction in intensity may be used to monitor the binding processes.

The invention will now be described in more detail, by way of illustration only, with reference to the accompanying drawings in which:
Figure 1 is plan view of a sample cell according to the invention,
Figure 2 is an end elevation of the sample cell of Figure 1,
Figure 3 is a sectional view along III - III in Figure 1 showing the sample cell positioned within the housing of an analytical device,
Figure 4 is a view corresponding to Figure 3 of a second embodiment of sample cell according to the invention,
Figure 5 is a view corresponding to Figure 3 of a third embodiment,
Figure 6 is a view corresponding to Figure 3 of a fourth embodiment,
Figure 7 is a sectional view of a fifth embodiment in which a resonant optical biosensor is formed directly on the surface of a moulded prism which is snap-fitted to a housing, and
Figure 8 is a perspective view of a wafer used in the manufacture of the prism of Figure 7.

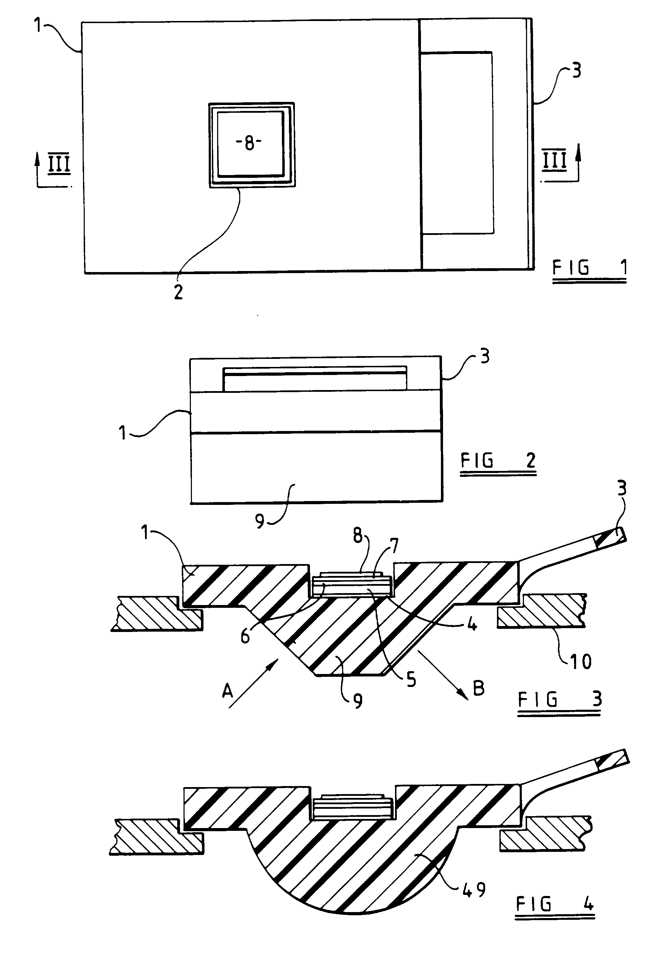

Referring first to Figures 1 to 3, a disposable sample cell for use in the determination of a biochemical analyte in solution comprises a moulded plastics block 1 defining a well 2 of square cross section. The block 1 is provided at one end with a handle 3 for ease of handling. As shown in Figure 3, located within the well 2 and bonded to the base of the well 2 by a layer of refractive index matching adhesive or fluid 4 is a resonant optical biosensor 5, 6, 7, 8.

The biosensor comprises a glass chip 5 approximately 10 mm square and having a thickness of approximately 1 mm. A spacer layer 6 of silicon dioxide is formed on the upper surface of the glass chip 5 and has a thickness of approximately 700nm. Formed on the spacer layer 6 is a cavity layer 7 of zirconium dioxide (approximate thickness 40nm). The lamella structure is completed by a layer of immobilised biochemical species 8, the nature of which depends on the assay technique to be performed. The thicknesses of the various layers are shown only schematically in the various Figures, and not to scale.

As can be seen from Figures 2 and 3, the lower portion of the block 1 is formed as a trapezoidal prism 9.

In use, the sample cell is located as shown in Figure 3 in an aperture in the housing 10 of an analytical apparatus containing appropriate irradiation and detection apparatus. A beam of monochromatic radiation from a laser (not shown) is incident upon the sample cell along the line of arrow A in Figure 3. The radiation is coupled into the biosensor by the prism 9 and is totally internally reflected at the interface between the glass chip 5 and the spacer layer 6. At a particular angle of incidence, the reflection is frustrated by resonant coupling of radiation into the cavity layer 7, which has a higher refractive index than either of the layers 6, 8 below and above it and therefore acts as a resonant cavity. At this angle the reflected radiation (arrow B in Figure 3) undergoes a phase change.

The phase of the reflected radiation is monitored as a function of angle by a suitable detector (not shown) and the angle at which resonance occurs determined. After a sample containing the analyte under test is contacted with the layer of immobilised biochemical species 8, the thickness and/or refractive index of that layer is altered, resulting in a change in the angular position of the resonance. The rate and/or extent of this change is monitored, providing a qualitative and quantitative indication of the presence of analyte in the sample.

Figure 4 shows an alternative embodiment in which the prism 49 is hemicylindrical.

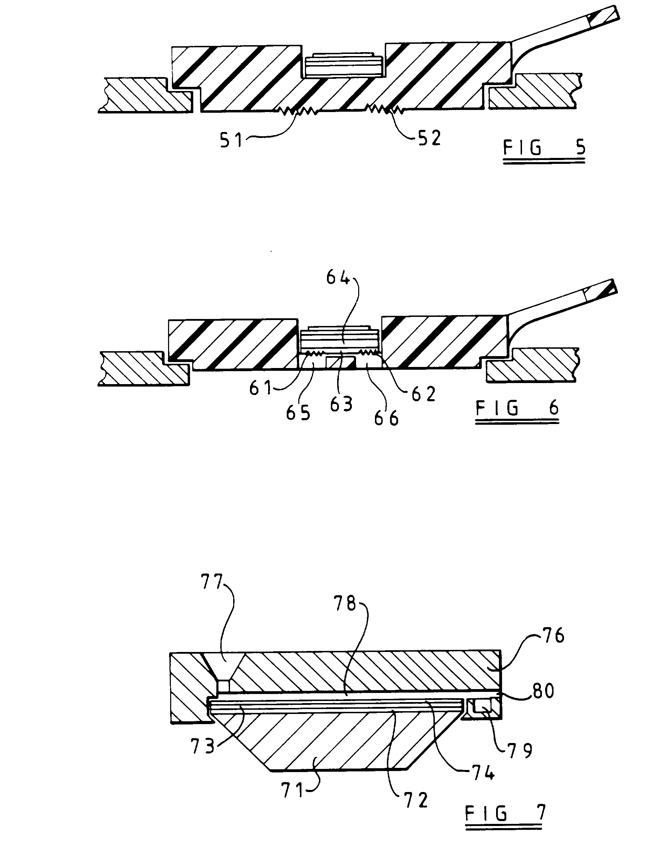

In the sample cell shown in Figure 5, radiation is coupled into and out of the sample cell by means of an input grating 51 and an output grating 52, both of which are formed in the under surface of the transparent block.

Figure 6 shows a further embodiment employing gratings to couple radiation into and out of the biosensor. This embodiment differs from that of Figure 5 in that the first and second gratings 61, 62 are provided not in the underside of the block but in a layer of film 63 affixed to the underside of the glass chip 64.

Apertures 65, 66 are provided in the block below the glass chip 65 to permit entry and exit of radiation.

In the embodiment of Figure 7, a trapezoidal prism 71 is moulded from clear plastics material and is coated with a layer of silicon dioxide 72 of approximate thickness 700 nm, a layer of zirconium dioxide 73 of approximate thickness 40nm and a layer of immobilised biochemicals 74. The layers 72, 73 constitute the spacer layer and cavity layer respectively of a resonant optical biosensor.

The coated prism 71 is snap-fitted into a plastics housing 76 in which are formed a sample entry port 77 connected to a capillary duct 78 formed between the upper surface of the coated prism 71 and the lower surface of the housing 76 which terminates at a sample trap 79. An air outlet 80 is provided in the sample trap 79.

In use, the assembled unit 71, 76 is located in an aperture in the housing of an appropriate apparatus. The lower surface of the spacer layer 72 is irradiated with a beam of monochromatic radiation from a laser (not shown), total internal reflection occurring. The angular position of the resonance is determined as described previously.

A patient's fingertip is punctured and a drop of blood expressed. The fingertip is inserted into the entry port 77 to transfer the drop of blood thereto. The blood is conducted along the duct 78 by capillary action from the entry port 77 across the surface of the coated prism 71, the excess sample being collected in the sample trap 79. The extent and/or rate of change of the angular position of resonance is monitored as described previously.

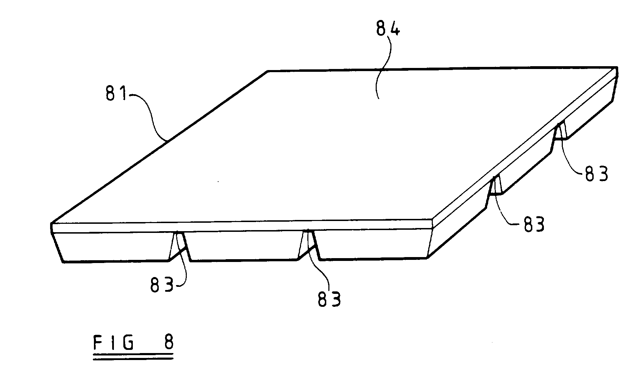

Figure 8 shows a wafer 81 of transparent prisms used in the manufacture of the block 71 of Figure 7. The wafer 81 is moulded as a generally square assembly of 3 x 3 trapezoidal prisms frangibly connected by relatively thin connecting sections 83 such that one surface 84 of the wafer 81 is generally flat. The layers 72, 73, 74 of material forming an optical biosensor are applied successively to the flat surface 84 and the individual prisms separated and fitted into housings 76.

## Claims

1. A sample cell for use in the qualitative or quantitative determination of an analyte, comprising a transparent block accommodating a resonant FTR-based optical biosensor, which biosensor comprises
a) a cavity layer of transmissive dielectric material of refractive index n₃,
b) a dielectric substrate of refractive index n₁, and
c) interposed between the cavity layer and the substrate, a dielectric spacer layer of refractive index n₂,
the arrangement being such that, when the interface between the substrate and the spacer layer is irradiated with light such that total reflection occurs, the total reflection may be frustrated by the propagation of a resonant guided mode within the cavity layer, and the transparent block having formed integrally therein optical coupling means to couple light into the biosensor.

2. A sample cell according to claim 1, wherein the optical biosensor is accommodated at an upper portion of the block and the optical coupling means is formed integrally in a lower portion of the block.

3. A sample cell according to claim 1 or claim 2, which is a disposable unit.

4. A sample cell according to any one of the preceding claims, in which the transparent block is of clear plastics material.

5. A sample cell according to any one of the preceding claims, in which the optical biosensor comprises a thin-film cavity layer of dielectric material.

6. A sample cell according to any one of the preceding claims, in which the optical coupling means is a prism.

7. A sample cell according to any one of claims 1 to 5, in which the optical coupling means is a grating.

8. A sample cell according to any one of the preceding claims, in which the transparent block is, or is part of, a moulded article.

9. A sample cell according to claim 8, in which the moulded article constitutes the substrate of the optical biosensor.

10. A sample cell according to any one of the preceding claims, which comprises a moulded transparent block upon which are coated the spacer layer and cavity layer of a resonant optical biosensor, the block fitting a housing in which is formed a sample entry port, a sample chamber or duct being defined between the block and the housing.

11. A method of manufacturing a sample cell according to any one of the preceding claims, which method comprises the steps of
a) moulding from a plastics material of refractive index n₁ a wafer comprising a plurality of transparent blocks frangibly connected at their edges,
b) applying a layer of dielectric material of refractive index n₂ of thickness 200 to 2000nm to the surface of the wafer, n₂ being lower than n₁
c) applying a layer of dielectric material of refractive index n₃, n₃ being greater than n₂, to the same surface of the wafer, and
d) separating the individual transparent blocks.

12. A method according to claim 11, in which the blocks are in the form of prisms.

## Patentansprüche

1. Probenzelle zur Verwendung bei der qualitativen oder quantitativen Bestimmung eines Analyten, mit einem lichtdurchlässigen Block, der einen auf dem Prinzip der verhinderten Totalreflexion (FTR) basierenden optischen Resonanz-Biosensor aufnimmt, wobei der Biosensor aufweist:
a) eine Hohlraumschicht aus lichtdurchlässigem dielektrischem Material mit einer Brechzahl n₃.
b) ein dielektrisches Substrat mit einer Brechzahl n₁, und
c) eine zwischen der Hohlraumschicht und dem Substrat eingesetzte dielektrische Abstandsschicht mit einer Brechzahl n₂,
wobei die Anordnung so gewählt ist, daß bei einer zur Totalreflexion führenden Lichteinstrahlung auf die Grenzfläche zwischen Substrat und Abstandsschicht die Totalreflexion durch die Ausbreitung einer geführten Resonatormode innerhalb der Hohlraumschicht verhindert werden kann,
und wobei in dem lichtdurchlässigen Block eine optische Kopplungseinrichtung zum Einkoppeln von Licht in den Biosensor integriert ist.

2. Probenzelle nach Anspruch 1, wobei der optische Biosensor in einem oberen Abschnitt des Blocks untergebracht ist und die optische Kopplungseinrichtung in einem unteren Abschnitt des Blocks integriert ist.

3. Probenzelle nach Anspruch 1 oder 2, die eine Einwegeinheit ist.

4. Probenzelle nach einem der vorhergehenden Ansprüche, wobei der lichtdurchlässige Block aus durchsichtigem Kunststoff besteht.

5. Probenzelle nach einem der vorhergehenden Ansprüche, wobei der optische Biosensor eine als Dünnschicht ausgebildete Hohlraumschicht aus dielektrischem Material aufweist.

6. Probenzelle nach einem der vorhergehenden Ansprüche, wobei die optische Kopplungseinrichtung ein Prisma ist.

7. Probenzelle nach einem der Ansprüche 1 bis 5, wobei die optische Kopplungseinrichtung ein Gitter ist.

8. Probenzelle nach einem der vorhergehenden Ansprüche, wobei der lichtdurchlässige Block ein Formkörper oder Teil eines Formkörpers ist.

9. Probenzelle nach Anspruch 8, wobei der Formkörper das Substrat des optischen Biosensors bildet.

10. Probenzelle nach einem der vorhergehenden Ansprüche, die einen geformten lichtdurchlässigen Block aufweist, auf dem die Abstandsschicht und die Hohlraumschicht eines optischen Resonanz-Biosensors aufgebracht sind, wobei der Block in ein Gehäuse paßt, in dem eine Probeneintrittsöffnung, eine Probenkammer oder ein Probenkanal zwischen dem Block und dem Gehäuse abgegrenzt sind.

11. Verfahren zur Herstellung einer Probenzelle nach einem der vorhergehenden Ansprüche, wobei das Verfahren die folgenden Schritte aufweist:
a) Formen eines Wafers mit mehreren lichtdurchlässigen Blöcken, die an ihren Kanten zerbrechbar miteinander verbunden sind, aus einem Kunststoff mit einer Brechzahl n₁,
b) Aufbringen einer Schicht aus dielektrischem Material mit einer Brechzahl n₂ und einer Dicke von 200 bis 2000 nm auf die klaferoberfläche, wobei n₂ kleiner als n₁ ist,
c) Aufbringen einer Schicht aus dielektrischem Material mit einer Brechzahl n₃, wobei n₃ größer als n₂ ist, auf die gleiche Waferoberfläche, und
d) Trennen der einzelnen lichtdurchlässigen Blöcke voneinander.

12. Verfahren nach Anspruch 11, wobei die Blöcke die Form von Prismen haben.

## Revendications

1. Cellule échantillon devant être utilisée pour la détermination qualitative ou quantitative d'un analyte, comprenant un bloc transparent contenant un biocapteur optique résonnant à base de FTR (réflexion totale diminuée), le dit biocapteur comprenant
a) une couche à cavité de matériau diélectrique de transmission d'indice de réfraction n₃,
b) un substrat diélectrique d'indice de réfraction n₁, et
c) intercalée entre la couche à cavité et le substrat, une couche intercalaire diélectrique d'indice de réfraction n₂,
l'agencement étant tel que, lorsque l'interface entre le substrat et la couche intercalaire est irradiée de lumière de sorte à provoquer une réflexion totale, la réflexion totale peut être diminuée par la propagation d'un mode guidé résonnant dans la couche à cavité,
et le bloc transparent ayant, faisant partie intégrante avec lui, un moyen de couplage optique destiné à coupler la lumière dans le biocapteur.

2. Cellule échantillon selon la revendication 1, où le biocapteur optique est logé dans une partie supérieure du bloc et le moyen de couplage optique fait partie intégrante d'une partie inférieure du bloc.

3. Cellule échantillon selon la revendication 1 ou la revendication 2, qui est une unité à usage unique.

4. Cellule échantillon selon l'une quelconque des revendications précédentes, où le bloc transparent est en matériau plastique transparent.

5. Cellule échantillon selon l'une quelconque des revendications précédentes, où le biocapteur optique comprend une couche à cavité de mince pellicule de matériau diélectrique.

6. Cellule échantillon selon l'une quelconque des revendications précédentes, où le moyen de couplage optique est un prisme.

7. Cellule échantillon selon l'une quelconque des revendications 1 à 5, où le moyen de couplage optique est une grille.

8. Cellule échantillon selon l'une quelconque des revendications précédentes, où le bloc transparent est un article moulé, ou en fait partie.

9. Cellule échantillon selon la revendication 8, où l'article moulé constitue le substrat du biocapteur optique.

10. Cellule échantillon selon l'une quelconque des revendications précédentes, qui comprend un bloc transparent moulé sur lequel sont revêtues la couche intercalaire et la couche à cavité d'un biocapteur optique résonnant, le bloc s'encastrant dans un boîtier dans lequel est formé un orifice d'introduction d'échantillon, une chambre ou un conduit d'échantillonnage délimité(e) entre le bloc et le boîtier.

11. Procédé de fabrication d'une cellule échantillon selon l'une quelconque des revendications précédentes, le dit procédé comprend les étapes suivantes
a) moulage à partir d'un matériau plastique d'indice de réfraction n₁ d'une tranche comprenant une multiplicité de blocs transparents raccordés de façon détachable en leurs bords,
b) application d'une couche de matériau diélectrique d'indice de réfraction n₂ d'une épaisseur de 200 à 2000 nm sur la surface de la tranche, n₂ étant inférieur à n₁,
c) application d'une couche de matériau diélectrique d'indice de réfraction n₃, n₃ étant supérieur à n₂, sur la même surface de tranche, et
d) séparation des blocs transparents individuels.

12. Procédé selon la revendication 11, où les blocs ont la forme de prismes.
